# EUROPÄISCHE PATENTSCHRIFT

(11) **EP 4 475 948 B1**
(45) Veröffentlichungstag und Bekanntmachung des Hinweises auf die Patenterteilung: **04.11.2026**
(21) Anmeldenummer: 23706279.9
(22) Anmeldetag: 30.01.2023
(51) Int. Cl.: A61N 5/06, G02B 5/12, G02B 5/20

(54) **RÜCKSTRAHLEINRICHTUNG FÜR BIOPHOTON/ZELLREAKTIVIERUNG**
REFLECTOR FOR BIOPHOTON/CELL REACTIVATION
RÉFLECTEUR POUR RÉACTIVATION DE BIOPHOTONS/CELLULES

(30) Priorität: 08.02.2022 DE 202022100700 U
(43) Veröffentlichungstag der Anmeldung: 18.12.2024
(73) Patentinhaber: Theralogy International GmbH, 79761 Waldshut-Tiengen (DE)
(72) Erfinder: OVERBECK, David, 79761 Waldshut-Tiengen (DE)
(74) Vertreter: Ostermann, Thomas
(86) Internationale Anmeldenummer: PCT/EP2023/052119
(87) Internationale Veröffentlichungsnummer: WO 2023/151972

(56) Entgegenhaltungen:
- EP-A1- 3 569 288
- WO-A1-2015/131885
- DE-A1- 19 904 315
- DE-U1- 202008 011 806
- DE-U1- 202022 100 700

## Beschreibung

Die Erfindung betrifft eine Rückstrahleinrichtung nach dem Oberbegriff des Patentanspruchs 1.

Aus der DE 20 2008 011 806 U1 ist eine Rückstrahleinrichtung für eine Biophotonentherapie bekannt, die einen Tragkörper, eine Reflexionsschicht und eine Filterschicht umfasst. Die Reflexionsschicht schließt sich an einer Aufnahmefläche des Trägerkörpers an. Die Filterschicht ist auf einer zu der Aufnahmefläche des Trägerkörpers gegenüberliegenden Seite der Reflexionsschicht angeordnet. Der topfförmige Trägerkörper wird durch eine Glasschicht abgedeckt, die auf der Filterschicht aufliegt. Nachteilig an der bekannten Rückstrahleinrichtung ist, dass sie aufgrund der Glasabdeckung relativ voluminös und schwer ist.

Aus der DE 199 04 315 A1 ist eine Rückstrahleinrichtung mit einer Reflexionsschicht bekannt, die zwischen zwei Folien klebend eingefasst ist. Die Rückstrahleinrichtung ist somit pflasterartig ausgebildet.

Aus der EP 3 569 288 A1 ist eine Rückstrahleinrichtung bekannt, die einen Tragkörper aufweist mit einer oberen Außenhülle und einer unteren

Außenhülle. Die Reflexionsschicht ist somit von dem Tragkörper vollkommen eingeschlossen.

Aus der WO 2015/131885 A1 ist eine Rückstrahleinrichtung mit einem Tragkörper bekannt, der einen Rand mit einer umlaufenden Schmalfläche aufweist. Der Rand begrenzt eine Aufnahmefläche, in der eine Reflexionsschicht und eine Filterschicht angeordnet sind. Die Schmalfläche des Randes verläuft somit bündig zu einer auf der Reflexionsfläche abgewandten Seite angeordneten Oberfläche der Filterschicht.

Aufgabe der vorliegenden Erfindung ist es, eine Rückstrahleinrichtung für Biophotonen/Zellreaktivierung derart weiterzubilden, dass die Handhabbarkeit vereinfacht und die Effektivität verbessert wird.

Zur Lösung dieser Aufgabe weist die Erfindung die Merkmale des Patentanspruchs 1 auf.

Nach der Erfindung ist eine Oberseite der Rückstrahleinrichtung durch eine erste Filterschicht oder eine zweite Filterschicht gebildet, die sich dadurch unterscheiden, dass sie Strahlung in einem unterschiedlichen Spektralbereich hindurchlassen. Vorteilhaft kann hierdurch die Variabilität der Rückstrahleinrichtung verbessert werden. Dadurch, dass die Rückstrahleinrichtung im Wesentlichen durch die optisch wirksame Filterschicht und Reflexionsschicht besteht (ohne zusätzliche Glasschicht), weist sie ein relativ geringes Gewicht auf und ist leicht handhabbar.

Nach einer bevorzugten Ausführungsform der Erfindung ist die erste Filterschicht derart ausgebildet, dass die Strahlung in einem Spektralbereich mit einer Wellenlänge zwischen 430 nm und 490 nm hindurchgelassen wird. Hierdurch wird Licht blauer Farbe hindurchgelassen.

Nach einer Weiterbildung der Erfindung ist die zweite Filterschicht derart ausgebildet, dass die Strahlung in einem Spektralbereich mit einer Wellenlänge zwischen 640 nm und 780 nm hindurchgelassen wird. Hierdurch wird Licht roter Farbe hindurchgelassen.

Nach einer bevorzugten Ausführungsform der Erfindung weisen die erste Filterschicht und die zweite Filterschicht eine gleiche Wanddicke auf. Die Rückstrahleinrichtung weist somit unabhängig von dem Typ der Filterschicht eine gleiche Dimension auf, so dass sie einfach herstellbar und leicht anzuwenden ist.

Nach einer Weiterbildung der Erfindung ist die Reflexionsschicht als eine Folie ausgebildet, die verglichen zu der ersten und zweiten Filterschicht eine wesentlich kleinere Dicke aufweist. Vorteilhaft kann hierdurch die Rückstrahleinrichtung relativ flach aufgebaut werden.

Nach einer bevorzugten Ausführungsform der Erfindung ist eine Deckseite der Rückstrahleinrichtung mit einer Haftungsschicht verbindbar, so dass die Rückstrahleinrichtung mit der Deckseite an ein Lebewesen lösbar befestigbar ist. Durch das relativ geringe Gewicht der Rückstrahleinrichtung wird einem unerwünschten Lösen der Rückstrahleinrichtung von einer Haut des Lebewesens entgegengewirkt.

Ein Ausführungsbeispiel der Erfindung wird nachfolgend anhand eines Ausführungsbeispiels näher erläutert.

Die Zeichnungen zeigen:

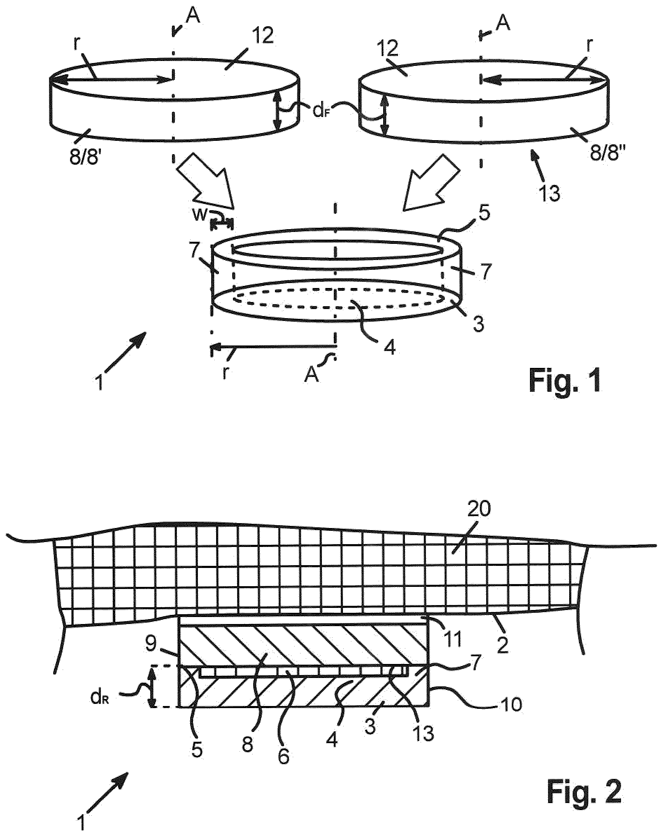

- Figur 1: eine perspektivische Darstellung einer Rückstrahleinrichtung ohne Reflexionsschicht und ohne Haftschicht und
- Figur 2: einen Querschnitt durch die Rückstrahleinrichtung in einem Anwendungszustand.

Eine erfindungsgemäße Rückstrahleinrichtung 1 dient zur Anlage an einer Haut 2 eines Lebewesens 20: Sie ermöglicht die Reaktivierung von Zellen im Rahmen eines Zelltrainings (Biophotonen/Zellreaktivierung). Bei den Lebewesen 20 kann es sich beispielsweise um Menschen oder Tiere handeln.

Die Rückstrahleinrichtung 1 weist einen Tragkörper 3 auf, der vorzugsweise aus einem nichttransparenten Material besteht. Der Tragkörper 3 ist tellerförmig ausgebildet mit einer Aufnahmefläche 4 und einer randseitigen Schmalfläche 5. Die randseitige Schmalfläche 5 ist im Vergleich zu der Aufnahmefläche 4 des Tragkörpers 3 erhaben ausgebildet. Die Erhabenheit der randseitigen Schmalfläche 5 im Vergleich zu der Aufnahmefläche 4 ist derart groß gewählt, dass eine Reflexionsschicht 6 von einem die Schmalfläche 5 aufweisenden Rand 7 des Tragkörpers 3 umgeben ist. Vorzugsweise ist die Reflexionsschicht 6 an eine Innenkontur des Randes 7 des Tragkörpers 3 angepasst, so dass ein Querverschieben der Reflexionsschicht 6 senkrecht zu einer Symmetrieachse A des Tragkörpers 3 relativ zum dem Tragkörper 3 verhindert wird.

Im eingesetzten Zustand der Reflexionsschicht 6 kann diese bündig zu der Schmalfläche 5 des Randes 7 verlaufen oder alternativ im Vergleich zu der randseitigen Schmalfläche 5 vertieft angeordnet sein.

Vorzugsweise ist der Tragkörper 3 rotationssymmetrisch ausgebildet. Im vorliegenden Ausführungsbeispiel ist die Rückstrahleinrichtung 1 bzw. der Tragkörper 3 zylinderförmig und/oder im Querschnitt kreisförmig ausgebildet.

Auf einer der Aufnahmefläche 4 des Tragkörpers 3 abgewandten Seite der Reflexionsschicht 6 schließt sich eine Filterschicht 8 an.

Die Filterschicht 8 kann wahlweise als eine erste Filterschicht 8' oder als eine zweite Filterschicht 8"ausgebildet sein, die sich dadurch unterscheiden, dass die erste Filterschicht 8' eine Strahlung in einem ersten Spektralbereich und die zweite Filterschicht 8" eine Strahlung in einem zweiten Spektralbereich durchlässt. Die Abmessung der ersten Filterschicht 8' und der zweiten Filterschicht 8" sind identisch.

Im vorliegenden Ausführungsbeispiel ist die erste Filterschicht 8' derart ausgebildet, dass Strahlung in einem Spektralbereich mit einer Wellenlänge zwischen 430 nm und 490 nm hindurchgelassen wird. Die zweite Filterschicht 8" ist derart ausgebildet, dass Strahlung in einem Spektralbereich mit einer Wellenlänge zwischen 640 nm und 780 nm hindurchgelassen wird.

Die Filterschicht 8 bzw. die erste Filterschicht 8' und die zweite Filterschicht 8" sind plattenförmig bzw. zylinderförmig ausgebildet, wobei eine Wanddicke d_{F} der Filterschicht 8, 8', 8" in einem Bereich zwischen 1 mm und 3 mm liegen kann. Ein Radius r der Filterschicht 8, 8', 8" liegt in einem Bereich zwischen 1 cm und 3 cm, vorzugsweise in einem Bereich zwischen 1,8 cm und 2,2 cm, insbesondere 2 cm.

Die Wanddicke d_{F} der Filterschicht 8, 8', 8" kann im Bereich einer Wanddicke d_{R} des Randes 7 des Tragkörpers 3 liegen.

Im vorliegenden Ausführungsbeispiel ist der Radius r der Filterschicht 8, 8', 8" gleich zu einem Radius r des Tragkörpers 3 bzw. des Randes 7 desselben. Im montierten Zustand liegt somit die Filterschicht 8, 8', 8" auf der Schmalfläche 5 des Randes 7 auf, wobei eine umlaufende Umfangsfläche 9 der Filterschicht 8 bündig zu einer Umfangsfläche 10 des Tragkörpers 3 verläuft. Die Rückstrahleinrichtung 1 ist somit zylinderförmig ausgebildet.

Auf einer der Reflexionsschicht 6 abgewandten Flachseite der Filterschicht 8, 8', 8" ist diese für eine Lichtstrahlung bzw. für insbesondere eine Biophotonenstrahlung transparenten Haftschicht 11 versehen, mittels derer der Tragkörper 3 an der Haut 2 des Lebewesens 20 befestigbar ist. Vorzugsweise ist die Haftschicht 11 als eine bioverträgliche Klebeschicht bzw. Klebefolie ausgebildet.

Nach einer nicht dargestellten alternativen Ausführungsform der Erfindung kann auf die Haftschicht 11 verzichtet werden. Zur Befestigung der Rückstrahleinrichtung 1 auf der Haut 2 sind dann andere Befestigungsmittel, beispielsweise ein im Medizinbereich verwendbares handelsübliches Klebeband, erforderlich.

Bezüglich der Haftschicht 11 wird in Kauf genommen, dass mit erhöhter Anzahl der Therapieanwendungen der Rückstrahleinrichtung 1 die Klebewirkung nachlässt. Um die Haftung der Rückstrahleinrichtung 1 an der Haut 2 zu gewährleisten, sind dann entsprechende Klebebänder erforderlich.

Nach einer nicht dargestellten Ausführungsform der Erfindung kann die Reflexionsschicht 6 auch auf einer der Aufnahmefläche 4 zugewandten Flachseite 13 der Filterschicht 8, 8', 8" aufgebracht sein, beispielsweise aufgedampft oder auflaminiert oder auflackiert.

Während der Anwendung bildet die auf der Reflexionsschicht 6 abgewandten Seite der Filterschicht 8, 8', 8" angeordnete Flachseite 12 als Deckseite der Rückstrahleinrichtung 1 die Anlagefläche zur Auflage unmittelbar auf der Haut 2 des Lebewesens. Während der Therapieanwendung passieren die Biophotonen die erste Filterschicht 8' und/oder die zweite Filterschicht 8" und werden an der Reflexionsschicht 6 reflektiert, so dass sie über die erste Filterschicht 8' und/oder zweite Filterschicht 8" wieder auf die Haut 2 des Lebewesens 20 treffen.

Eine Wandbreite w der Schmalfläche 5 liegt in einem Bereich zwischen 2 mm und 6 mm, vorzugsweise im Bereich von 3 mm bis 5 mm. Auf diese Weise ist gewährleistet, dass die Filterschicht 8, 8', 8" haftend an dem Tragkörper 3 befestigbar ist. Die Befestigung der Filterschicht 8 an dem Tragkörper 3 erfolgt vorzugsweise durch Verklebung.

## Patentansprüche

1. Rückstrahleinrichtung für Biophotonen/Zellreaktivierung mit einem Tragkörper (3) enthaltend eine Aufnahmefläche (4), mit einer sich an die Aufnahmefläche (4) des Tragkörpers (3) anschließenden Reflexionsschicht (6) und mit einer sich auf einer der Aufnahmefläche (4) abgewandten Seite der Reflexionsschicht (6) sich anschließenden Filterschicht (8, 8', 8"), wobei die Filterschicht (8) wahlweise durch eine erste Filterschicht (8') oder durch eine zweite Filterschicht (8") gebildet ist, die jeweils eine Strahlung in einem unterschiedlichen Spektralbereich hindurchlassen, und wobei die erste Filterschicht (8, 8') und die zweite Filterschicht (8, 8") plattenförmig und/oder zylinderförmig ausgebildet sind; **dadurch gekennzeichnet, dass** ein Radius (r) der Filterschicht (8, 8', 8") einem Radius (r) des Tragkörpers (3) entspricht, wobei die Filterschicht (8, 8', 8") im montierten Zustand auf der Schmalfläche (5) des Randes (7) des Tragkörpers (3) aufliegt und wobei eine umlaufende Umfangsfläche (9) der Filterschicht (8, 8', 8") bündig zu einer Umfangsfläche (10) des Tragkörpers (3) verläuft, und dass das Filterelement (8, 8', 8") haftend an dem Tragkörper (3) befestigt ist.

2. Rückstrahleinrichtung nach Anspruch 1, wobei
die erste Filterschicht (8, 8') derart ausgebildet ist, dass Strahlung in einem Spektralbereich mit einer Wellenlänge zwischen 430 nm und 490 nm hindurchgelassen wird.

3. Rückstrahleinrichtung nach Anspruch 1 oder 2 wobei
die zweite Filterschicht (8, 8") derart ausgebildet ist, dass Strahlung in einem Spektralbereich mit einer Wellenlänge zwischen 640 nm und 780 nm hindurchgelassen wird.

4. Rückstrahleinrichtung nach einem der Ansprüche 1 bis wobei
die erste Filterschicht (8, 8') und die zweite Filterschicht (8, 8") eine gleiche Wanddicke (d_{F}) aufweisen.

5. Rückstrahleinrichtung nach einem der Ansprüche 1 bis 4, wobei
die Wanddicke (d_{F}) der ersten Filterschicht (8') und der zweiten Filterschicht (8") im Bereich von 2 mm bis 10 mm, vorzugsweise 1 mm bis 3 mm, ist.

6. Rückstrahleinrichtung nach einem der Ansprüche 1 bis 5, wobei
die Reflexionsschicht (6) als eine weißfarbene Papierscheibe oder weißfarbene Kunststoffscheibe ausgebildet ist.

7. Rückstrahleinrichtung nach einem der Ansprüche 1 bis 6, wobei
die Reflexionsschicht (6) auf einer Flachseite (13) der ersten Filterschicht (8') und der zweiten Filterschicht (8") aufgedampft oder auflaminiert oder auflackiert ist.

8. Rückstrahleinrichtung nach einem der Ansprüche 1 bis 7, wobei
eine auf der Reflexionsschicht (6) abgewandten Seite angeordneten Flachseite (12) der Filterschicht (8, 8', 8") derart ausgebildet ist, dass sie mit einer lichttransparenten oder biophotonentransparenten Haftschicht (11) verbindbar ist zur Befestigung der Rückstrahleinrichtung (1) an einer Haut (2) eines Lebewesens (20).

9. Rückstrahleinrichtung nach Anspruch 8, wobei die Haftschicht (11) als eine bioverträgliche Klebefolie ausgebildet.

## Claims

1. A reflector for biophoton/cell reactivation with a support body (3) containing a receiving surface (4), with a reflective layer (6) adjoining the receiving surface (4) of the support body (3) and with a filter layer (8, 8', 8") adjoining a side of the reflective layer (6) facing away from the receiving surface (4), wherein the filter layer (8) is formed selectively by a first filter layer (8') or by a second filter layer (8") which each allow radiation in a different spectral range to pass through, and wherein
the first filter layer (8, 8') and the second filter layer (8, 8") have a plate-shaped and/or cylindrical configuration, **characterized in that** a radius (r) of the filter layer (8, 8', 8") corresponds to a radius (r) of the support body (3), wherein, in the assembled state, the filter layer (8, 8', 8") rests on the narrow surface (5) of the rim (7) of the support body (3) and wherein a continuous circumferential surface (9) of the filter layer (8, 8', 8") runs flush with a circumferential surface (10) of the support body (3), and **in that** the filter element (8, 8', 8") is fastened to the support body (3) in an adhesive manner.

2. The reflector according to Claim 1, wherein the first filter layer (8, 8') is configured such that radiation in a spectral range with a wavelength between 430 nm and 490 nm is allowed to pass through.

3. The reflector according to Claim 1 or 2, wherein the second filter layer (8, 8") is configured such that radiation in a spectral range with a wavelength between 640 nm and 780 nm is allowed to pass through.

4. The reflector according to any one of Claims 1 to 3, wherein the first filter layer (8, 8') and the second filter layer (8, 8") have the same wall thickness (d_{F}).

5. The reflector according to any one of Claims 1 to 4, wherein the wall thickness (d_{F}) of the first filter layer (8') and the second filter layer (8") is in the range from 2 mm to 10 mm, preferably 1 mm to 3 mm.

6. The reflector according to any one of Claims 1 to 5, wherein the reflective layer (6) is configured as a white-colored paper disk or white-colored plastic disk.

7. The reflector according to any one of Claims 1 to 6, wherein the reflective layer (6) is vapor-deposited, laminated or painted onto a flat side (13) of the first filter layer (8') and the second filter layer (8").

8. The reflector according to any one of Claims 1 to 7, wherein a flat side (12) of the filter layer (8, 8', 8") arranged on the side facing away from the reflective layer (6) is configured such that it can be connected to a light-transparent or biophoton-transparent adhesive layer (11) for fastening the reflector (1) to the skin (2) of a living being (20).

9. The reflector according to Claim 8, wherein the adhesive layer (11) is configured as a biocompatible adhesive film.

## Revendications

1. Dispositif de réflexion de biophotons/de réactivation cellulaire comprenant un corps support (3) contenant une surface de réception (4), une couche réfléchissante (6) raccordée à la surface de réception (4) du corps support (3), et une couche filtrante (8, 8', 8") raccordée à un côté de la couche réfléchissante (6) détourné de la surface de réception (4), la couche filtrante (8) étant formée, au choix, par une première couche filtrante (8') ou par une deuxième couche filtrante (8"), qui laissent respectivement passer un rayonnement dans une plage spectrale différente, et la première couche filtrante (8, 8') et la deuxième couche filtrante (8, 8") étant réalisées sous forme de plaque et/ou sous forme de cylindre ; **caractérisé en ce qu'**un rayon (r) de la couche filtrante (8, 8', 8") correspond à un rayon (r) du corps support (3), la couche filtrante (8, 8', 8") reposant, à l'état monté, sur la surface étroite (5) du bord (7) du corps support (3), et une surface périphérique circonférentielle (9) de la couche filtrante (8, 8', 8") étant affleurante à une surface périphérique (10) du corps support (3), et **en ce que** l'élément filtrant (8, 8', 8") est fixé par adhérence au corps support (3).

2. Dispositif de réflexion selon la revendication 1, la première couche filtrante (8, 8') étant réalisée de manière à laisser passer un rayonnement dans une plage spectrale présentant une longueur d'onde comprise entre 430 nm et 490 nm.

3. Dispositif de réflexion selon la revendication 1 ou 2, la deuxième couche filtrante (8, 8") étant réalisée de manière à laisser passer un rayonnement dans une plage spectrale présentant une longueur d'onde comprise entre 640 nm et 780 nm.

4. Dispositif de réflexion selon l'une quelconque des revendications 1 à 3, la première couche filtrante (8, 8') et la deuxième couche filtrante (8, 8") présentant une même épaisseur de paroi (d_{F}).

5. Dispositif de réflexion selon l'une quelconque des revendications 1 à 4, l'épaisseur de paroi (d_{F}) de la première couche filtrante (8') et de la deuxième couche filtrante (8") étant dans la plage de 2 mm à 10 mm, de préférence de 1 mm à 3 mm.

6. Dispositif de réflexion selon l'une quelconque des revendications 1 à 5, la couche réfléchissante (6) étant réalisée sous la forme d'un disque en papier blanc ou d'un disque en matière plastique blanche.

7. Dispositif de réflexion selon l'une quelconque des revendications 1 à 6, la couche réfléchissante (6) étant déposée par évaporation, laminée ou laquée sur un côté plan (13) de la première couche filtrante (8') et de la deuxième couche filtrante (8").

8. Dispositif de réflexion selon l'une quelconque des revendications 1 à 7, un côté plan (12) de la couche filtrante (8, 8', 8"), disposée sur le côté détourné de la couche réfléchissante (6), étant réalisé de manière à pouvoir être relié à une couche adhésive (11) transparente à la lumière ou transparente aux biophotons afin de fixer le dispositif de réflexion (1) sur une peau (2) d'un être vivant (20).

9. Dispositif de réflexion selon la revendication 8, la couche adhésive (11) étant réalisée sous la forme d'un film adhésif biocompatible.
